(19) Europäisches Patentamt
European Patent Office
Office européen des brevets

(11) Veröffentlichungsnummer: **0 056 938**
**B1**

(12) # EUROPÄISCHE PATENTSCHRIFT

(45) Veröffentlichungstag der Patentschrift: **27.03.85**

(21) Anmeldenummer: **82100119.5**

(22) Anmeldetag: **11.01.82**

(51) Int. Cl.⁴: **C 07 D 253/06,** C 07 D 405/04, C 07 D 409/04 // C07C103/133, C07C103/62, C07C103/80

(54) **Verfahren zur Herstellung von 3,6-disubstituierten 4-Amino-1,2,4-triazin-5-onen.**

(30) Priorität: **24.01.81 DE 3102318**

(43) Veröffentlichungstag der Anmeldung: **04.08.82 Patentblatt 82/31**

(45) Bekanntmachung des Hinweises auf die Patenterteilung: **27.03.85 Patentblatt 85/13**

(84) Benannte Vertragsstaaten: **AT BE CH DE FR GB IT LI NL**

(56) Entgegenhaltungen:
**DE - A - 2 165 554**
**DE - A - 2 556 835**
**DE - A - 2 856 750**

(73) Patentinhaber: **BAYER AG, Konzernverwaltung RP Patentabteilung, D-5090 Leverkusen 1 Bayerwerk (DE)**

(72) Erfinder: **Schmidt, Thomas, Dr., Am Bandenfeld 72, D-5657 Haan 1 (DE)**
Erfinder: **Timmler, Helmut, Dr., Tersteegenweg 16, D-5600 Wuppertal 11 (DE)**
Erfinder: **Bonse, Gerhard, Dr., Wolfskaul 3, D-5000 Köln 80 (DE)**
Erfinder: **Blank, Heinz Ulrich, Dr., Am Geusfelde 35, D-5068 Odenthal (DE)**
Erfinder: **Marzolph, Gerhard, Dr., Roggendorfstrasse 65, D-5000 Köln 80 (DE)**

**EP 0 056 938 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein neues, vorteilhaftes Verfahren zur Herstellung von weitgehend bekannten, herbizid wirksamen 3,6-disubstituierten 4-Amino-1,2,4-triazin-5-onen.

Es ist bereits bekannt geworden, daß man bestimmte 1,2,4-Triazin-5-one erhält, wenn man $\alpha$-Ketocarbonsäuren, deren Ester oder Nitrile mit Hydrazidinen oder deren Salzen in Gegenwart eines Lösungsmittels und gegebenenfalls in Gegenwart eines Säurebindemittels bei Temperaturen zwischen −10 und +100°C gemäß folgendem Reaktionsschema umsetzt (vgl. DE-OS 2 224 161 und DE-OS 2 556 835):

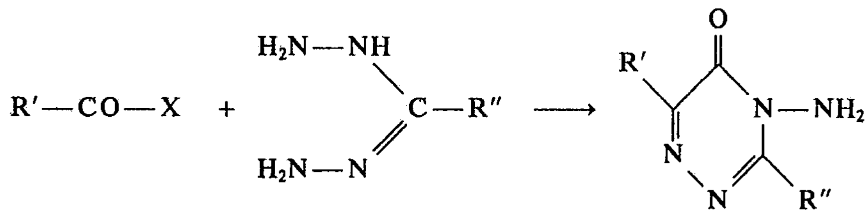

$R'$ = H, Alkyl, Cycloalkyl, Aralkyl, Aryl, Thienyl, Furyl;
$R''$ = Alkyl, Cycloalkyl, Aralkyl, Aryl;
$X$ = −COOH, −COOR‴, −CN;
$R'''$ = Alkyl, Aralkyl, Aryl.

Dieses Verfahren hat jedoch den Nachteil, daß sich die Nitrile (X = CN), wenn überhaupt, nur in sehr schlechten Ausbeuten umsetzen lassen und die Säuren bzw. Ester (X = −COOH bzw. −COOR‴) normalerweise durch Hydrolyse bzw. Alkoholyse in einer mehrstufigen Reaktion – über die Stufe des Amids – aus den Nitrilen hergestellt werden müssen.

Es ist ferner bekannt, daß man 1,2,4-Triazin-5-one, welche in 3-Stellung durch die Heteroatomgruppe »$-X-R_2$« substituiert sind, erhält, wenn man $\alpha$-Ketocarbonsäureamide mit Semicarbaziden, Thiosemicarbaziden bzw. Aminoguanidin-Derivaten in Gegenwart eines polaren Lösungsmittels und gegebenenfalls in Gegenwart eines sauren Katalysators bei Temperaturen zwischen −20° und +150°C gemäß folgendem Reaktionsschema umsetzt (vgl. DE-OS 2 165 554):

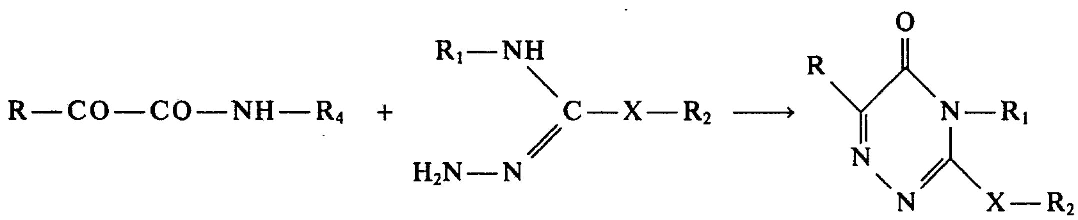

$R$ = Alkyl, Aralkyl, Aryl, (jeweils gegebenenfalls substituiert);
$R_1$ = aliphatischer Rest, Amino, Alkylamino, Dialkylamino;
$R_2$ = H, Alkyl;
$X$ = O, S, N($R_3$);
$R_3$ = H, Alkyl;
$R_4$ = z. B. Alkyl, Aryl (fehlt im Endprodukt; wird als Amin $R_4-NH_2$ abgespalten).

Dieses Verfahren ist jedoch nicht dazu geeignet, in 3-Stellung durch Kohlenwasserstoffreste substituierte 1,2,4-Triazin-5-one herzustellen.

Es wurde nun überraschend gefunden, daß man die weitgehend bekannten 3,6-disubstituierten (in 3-Stellung durch Kohlenwasserstoffreste substituierten) 4-Amino-1,2,4-triazin-5-one der Formel

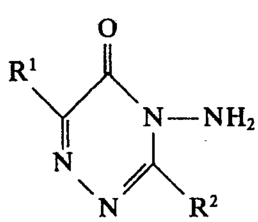 (I)

in welcher

$R^1$ für Wasserstoff, gegebenenfalls substituiertes Alkyl, gebenenenfalls substituiertes Cycloalkyl, gegebenenfalls substituiertes Cycloalkenyl, Alkenyl, Furyl, Thienyl oder gegebenenfalls substituiertes Aralkyl steht und

$R^2$ für Alkyl, gegebenenfalls substituiertes Cycloalkyl, gegebenenfalls substituiertes Aryl oder gegebenenfalls substituiertes Aralkyl steht,

in sehr guten Ausbeuten und in sehr reiner Form durch eine neue, zweistufige Cyclisierungsreaktion herstellen kann, indem man $\alpha$-Ketocarbonsäureamide der Formel

$$R^1-CO-CO-NHR^3 \qquad (II)$$

in welcher

$R^1$ die oben angegebene Bedeutung hat und
$R^3$ für Wasserstoff oder die Gruppe $-CO-R^4$ steht, wobei
$R^4$ für Alkyl, Cycloalkyl, gegebenenfalls substituiertes Aryl oder gegebenenfalls substituiertes Aralkyl steht,

pro Mol mit 1–1,5 Mol eines Hydrazidins der Formel

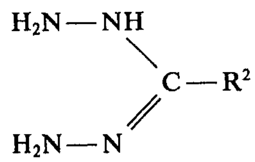

(III)

in welcher

$R^2$ die oben angegebene Bedeutung hat,

oder eines ihrer Säureadditionssalze, gegebenenfalls in Gegenwart eines Verdünnungsmittels, bei Temperaturen zwischen 0° und 150°C umsetzt, wobei in der ersten Verfahrensstufe eine Kondensationsreaktion zu noch offenkettigen Kondensationsprodukten und in der zweiten Verfahrensstufe eine Eliminierungsreaktion unter Ringschluß zu den Endprodukten (I) stattfindet.

Die erste Verfahrensstufe (Kondensationsreaktion) wird gegebenenfalls in Gegenwart eines sauren Katalysators und die zweite Verfahrensstufe (Eliminierungsreaktion unter Ringschluß) wird gegebenenfalls in Gegenwart einer Base durchgeführt, vorzugsweise ohne Zwischenisolierung der in der ersten Verfahrensstufe gebildeten Kondensationsprodukte der allgemeinen Formel

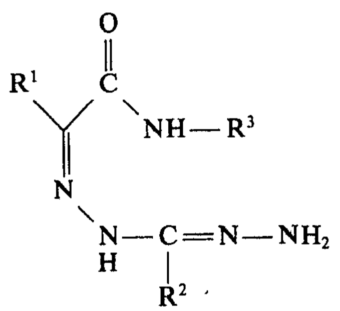

(IV a)

bzw.

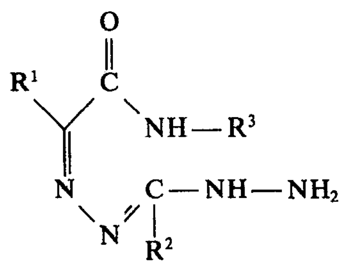

(IVb)

Es ist als ausgesprochen überraschend zu bezeichnen, daß sich die – im Vergleich zu den Ketocarbonsäuren oder deren Estern – reaktionsträgen Amide der Formel (II) sehr glatt und in hoher Ausbeute in der angegebenen Weise umsetzen lassen.

Das erfindungsgemäße Verfahren weist eine Reihe von Vorteilen auf. Insbesondere sind die als Ausgangsstoffe zu verwendenden $\alpha$-Ketocarbonsäureamide der Formel (II) leicht und in hoher Ausbeute und Reinheit aus den entsprechenden Nitrilen direkt herstellbar. Außerdem ist das erfindungsgemäße Verfahren breit anwendbar und führt kaum zur Bildung von Nebenprodukten. – Besonders zweckmäßig ist es, die Gesamtreaktion als »Eintopfverfahren« durchzuführen.

Die nach dem erfindungsgemäßen Verfahren herstellbaren 3,6-disubstituierten 4-Amino-1,2,4-triazin-5-one sind durch die Formel (I) allgemein definiert. In dieser Formel steht $R^1$ vorzugsweise für Wasserstoff; gegebenenfalls substituiertes geradkettiges oder verzweigtes Alkyl mit 1 bis 12 Kohlenstoffatomen, wobei als Substituenten beispielsweise genannt seien: Halogen (insbesondere Fluor und Chlor), Alkoxy und Alkylthio mit jeweils 1 bis 4 Kohlenstoffatomen; gegebenenfalls substituiertes Cycloalkyl mit 3 bis 6 Kohlenstoffatomen und gegebenenfalls substituiertes Cycloalkenyl mit 4 bis 6 Kohlenstoffatomen, wobei als Substituenten jeweils vorzugsweise genannt seien: Halogen (insbesondere Fluor und Chlor), Alkyl und Alkoxy mit jeweils 1 bis 4 Kohlenstoffatomen, sowie gegebenenfalls durch Halogen (insbesondere Fluor, Chlor und Brom) oder Alkyl mit 1 bis 4 Kohlenstoffatomen substituiertes Phenyl; Alkenyl mit 2 bis 6 Kohlenstoffatomen; Furyl und Thienyl; sowie für gegebenenfalls substituiertes Aryl und Aralkyl mit jeweils 6 bis 10 Kohlenstoffatomen im Arylteil und 1 bis 2 Kohlenstoffatomen im Alkylteil, wie insbesondere Phenyl und Benzyl, wobei als Substituenten jeweils vorzugsweise genannt seien: Halogen (insbesondere Fluor, Chlor und Brom); Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und bis zu 5 gleichen oder verschiedenen Halogenatomen (wie insbesondere Fluor- und Chloratomen); sowie Nitro.

$R^2$ steht vorzugsweise für geradkettiges oder verzweigtes Alkyl mit 1 bis 6 Kohlenstoffatomen; gegebenenfalls substituiertes Cycloalkyl mit 3 bis 6 Kohlenstoffatomen, wobei als Substituenten die bei $R^1$ bereits vorzugsweise genannten in Frage kommen; sowie für gegebenenfalls substituiertes Aryl und Aralkyl mit jeweils 1 bis 10 Kohlenstoffatomen im Arylteil und 1 bis 2 Kohlenstoffatomen im Alkylteil, wie insbesondere Phenyl und Benzyl, wobei als Substituenten jeweils die bereits bei $R^1$ vorzugsweise genannten in Frage kommen.

Besonders bevorzugt sind diejenigen Verbindungen der Formel (I), in denen $R^1$ für Isopropyl, sek.-Butyl, n-Butyl, tert.-Butyl, sek.-Pentyl, Neopentyl, 1,1-Dimethylpropyl, Dichlorcyclopropyl, Methyldichlorcyclopropyl, Cyclobutyl, Cyclopentyl, Cyclohexyl, Furyl, Thienyl, sowie für gegebenenfalls durch Fluor, Chlor, Brom, Methoxy, Nitro, Methyl und Trifluormethyl substituiertes Phenyl steht und $R^2$ für Methyl, Ethyl, n-Propyl, Isopropyl, n-Butyl, sek.-Butyl, tert.-Butyl, Cyclopropyl, Cyclobutyl oder Cyclopentyl steht.

Verwendet man beispielsweise Phenylglyoxylsäureamid und Acethydrazidin-hydrochlorid als Ausgangsstoffe, so kann der Reaktionsablauf durch das folgende zusammenfassende Formelschema wiedergegeben werden:

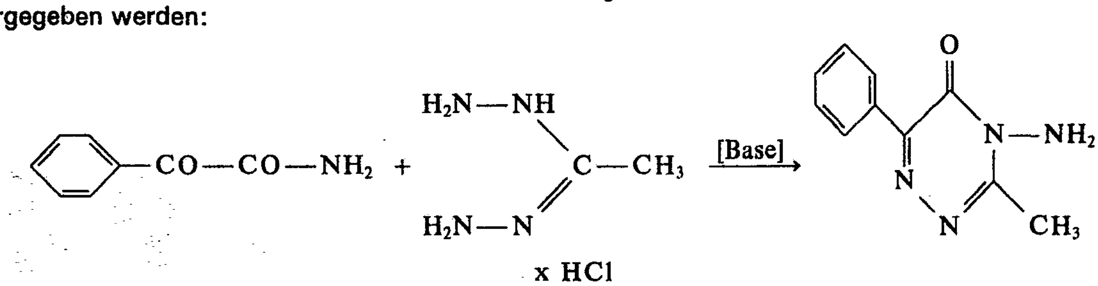


Als Zwischenprodukt tritt hierbei die folgende Verbindung auf:

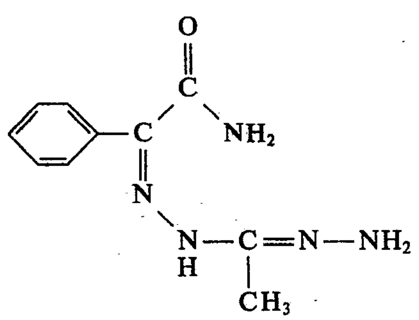

bzw.

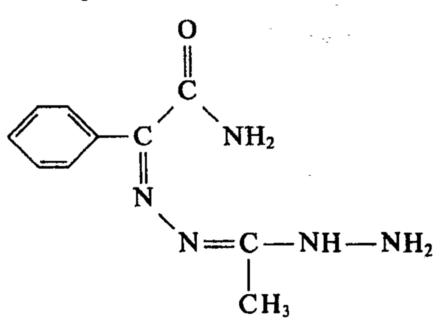

Die bei der Durchführung des erfindungsgemäßen Verfahrens als Ausgangsstoffe benötigten $\alpha$-Ketocarbonsäureamide sind durch die Formel (II) allgemein definiert. In dieser Formel steht $R^1$ vorzugsweise für diejenigen Reste, die bereits im Zusammenhang mit der Beschreibung der erfindungsgemäß herstellbaren Stoffe der Formel (I) vorzugsweise für diesen Substituenten genannt wurden. $R^3$ steht vorzugsweise für Wasserstoff und die Gruppe $-CO-R^4$, wobei $R^4$ vorzugsweise für Alkyl mit 1 bis 10 Kohlenstoffatomen, Cycloalkyl mit 3 bis 6 Kohlenstoffatomen sowie gegebenenfalls substituiertes Aryl und Aralkyl mit jeweils 6 bis 10 Kohlenstoffatomen im Arylteil und 1 bis 2 Kohlenstoffatomen im Alkylteil, wie insbesondere Phenyl und Benzyl steht, wobei als Substituenten jeweils die bei $R^1$ bereits vorzugsweise genannten in Frage kommen.

Die $\alpha$-Ketocarbonsäureamide der Formel (II) sind teilweise bekannt (vgl. z.B. Beilstein 10, 658; 10/I, 314; 10/II, 456 und 3, 620), teilweise sind sie Gegenstand eigener älterer Patentanmeldungen (vgl. Deutsche Patentanmeldungen P 3 003 542.8 vom 31. 1. 1980 (= EP-A-0 034 703) und P 3 009 044.9 vom 8. 3. 1980 (= EP-A-0 035 707). Sie können z B. durch partielle Hydrolyse entsprechender Nitril-Derivate, gegebenenfalls unter acylierenden Bedingungen, hergestellt werden (vgl. auch Herstellungsbeispiele). Als Beispiele seien im einzelnen genannt:

Phenylglyoxylsäureamid, Trimethylbrenztraubensäureamid, Phenylglyoxylsäure-N-acetyl-amid, Trimethylbrenztraubensäure-N-acetyl-amid, Cyclohexylglyoxylsäureamid, Cyclohexylglyoxylsäure-N-acetyl-amid, Dichlorcyclopropylglyoxylsäureamid, Dichlorcyclopropylglyoxylsäure-N-acetylamid.

Die außerdem bei der Durchführung des erfindungsgemäßen Verfahrens als Ausgangsstoffe benötigten Hydrazidine sind durch die Formel (III) allgemein definiert. In dieser Formel steht $R^2$ vorzugsweise für diejenigen Reste, die bereits im Zusammenhang mit der Beschreibung der erfindungsgemäß herstellbaren Stoffe der Formel (I) vorzugsweise für diesen Substituenten genannt wurden.

Wie bereits erwähnt, werden die Hydrazidine der Formel (III) vorzugsweise in Form von anorganischen oder organischen Säureadditionssalzen eingesetzt. Als Säurekomponente kommen alle üblicherweise verwendbaren starken anorganischen und organischen Säuren in Frage. Als Beispiele seien genannt: Chlorwasserstoffsäure, Bromwasserstoffsäure, Salpetersäure, Schwefelsäure, p-Toluolsulfonsäure, Phosphorsäure, Methansulfonsäure und Naphthalindisulfonsäure.

Die Hydrazidine der Formel (III) sowie ihre Säureadditionssalze sind allgemein bekannte Verbindungen der organischen Chemie, bzw. können sie in allgemein bekannter Art und Weise erhalten werden (vgl. hierzu z. B. Liebigs Ann. Chem. 1975, S. 1120–1123 und Monatshefte Chem. 63, S. 285–300 [1933]). Als Beispiele seien genannt:

Acethydrazidin, Propionylhydrazidin, n-Butyrylhydrazidin, Isopropionylhydrazidin, Cyclopropancarbonsäure-hydrazidin sowie deren Hydrochloride.

Als Verdünnungsmittel kommen für die erfindungsgemäße Umsetzung sowohl Wasser als auch inerte organische Lösungsmittel in Frage. Hierzu gehören vorzugsweise Ether, wie Tetrahydrofuran, Dioxan oder Ethylenglykolmonomethylether; Amide, wie Dimethylformamid, Diethylformamid, Dimethylacetamid oder N-Methylpyrrolidon; Alkohole, wie Methanol, Ethanol, n-Butanol, Isobutanol, tert.-Butanol, Glycol, Glycerin, n-Propanol, Isopropanol, Amylalkohol, Cyclohexanol, 2-Methyl-4-pentanol, Methylglykol, n-Hexanol, Isohexylalkohol, Isoheptylalkohol, n-Heptanol, Nonylalkohol, Dodecylalkohol oder Methylcyclohexanol; Kohlenwasserstoffe, wie Benzol oder Toluol; sowie halogenierte Kohlenwasserstoffe, wie Methylenchlorid, Chloroform, Tetrachlorkohlenstoff, 1,2-Dichlorethan, 1,1,2-Trichlorethan, Trichlorethylen sowie Chlorbenzol. Es können auch entsprechende Lösungsmittelgemische verwendet werden.

Die erste Stufe des erfindungsgemäßen Verfahrens kann mit Vorteil in Gegenwart eines sauren Katalysators durchgeführt werden, wobei dieser im allgemeinen in Mengen von etwa 0,05–0,1 Mol pro Mol (II) eingesetzt wird. Dies ist besonders in den Fällen zu empfehlen, in denen nicht die reaktiven $\alpha$-Ketocarbonsäure-N-acylamide, sondern die weniger reaktiven N-substituierten $\alpha$-Ketocarbonsäureamide als Ausgangsstoffe eingesetzt werden. Besonders bewährt hat sich als saurer Katalysator p-Toluolsulfonsäure.

Die zweite Stufe des erfindungsgemäßen Verfahrens muß dann in Gegenwart einer zumindest stöchiometrischen Menge einer Base durchgeführt werden, wenn man als Ausgangsprodukte die Hydrazidine (III) in Form von Säureadditionssalzen einsetzt. Aber auch im Falle, daß die Hydrazidine in freier Form verwendet werden, kann es zweckmäßig sein, bei dieser Stufe unter Zusatz einer katalytischen Menge einer Base zu arbeiten. – Als Basen können im Prinzip alle üblicherweise als Säurebindemittel verwendbaren anorganischen und organischen basischen Verbindungen eingesetzt werden. Hierzu gehören vorzugsweise Alkali- und Erdalkalimetalloxide, -hydroxide, -carbonate und -hydrogencarbonate, wobei als Metalle vorzugsweise in Frage kommen: Natrium, Kalium und Calcium; sowie tertiäre Amine, wie Trimethylamin, Triethylamin, Pyridin, Lauryldimethylamin, Stearyldimethylamin, N,N-Diethylcyclohexylamin, N-Ethylpiperidin, N-Methylpyrrolidin, $\alpha,\beta$- und $\gamma$-Picolin, N-Propylpiperidin, Chinolin, Isochinolin, Chinoxalin, Tri-n-amylamin, Tri-n-propylamin oder N,N-Dimethylbenzylamin.

Die Reaktionstemperaturen können bei der Durchführung des erfindungsgemäßen Verfahrens in einem größeren Bereich variiert werden. Im allgemeinen arbeitet man zwischen 0 und +150°C, vorzugsweise zwischen 10 und 120°C.

Die erfindungsgemäße Umsetzung kann unter Normaldruck oder bei erhöhtem Druck, kontinuierlich oder diskontinuierlich durchgeführt werden.

Bei der Durchführung des erfindungsgemäßen Verfahrens setzt man im allgemeinen auf 1 Mol $\alpha$-Ketocarbonsäureamid der Formel (II) 1–1,5 Mol, vorzugsweise 1–1,3 Mol eines Hydrazidins der Formel (III) bzw. eines entsprechenden Hydrazidin-Säureadditionssalzes und in der ersten Verfahrensstufe gegebenenfalls 0,05–0,1 Mol eines sauren Katalysators sowie in der zweiten Verfahrensstufe gegebenenfalls 1–3,5 Mol einer Base ein.

Die Isolierung der Endprodukte der Formel (I) erfolgt in üblicher Weise. Außerdem ist es möglich, die bei der ersten Stufe der erfindungsgemäßen Umsetzung auftretende stabile Zwischenstufe (Kondensationsprodukte) der Formel

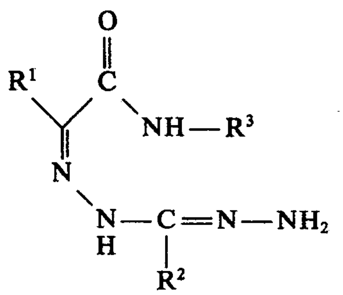

bzw.

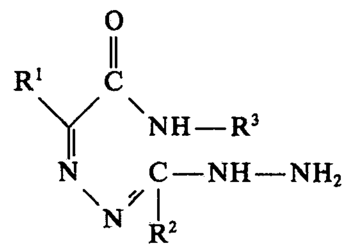

(IVb)

(IVa)

in welcher

$R^1$, $R^2$ und $R^3$ die oben angegebene Bedeutung haben,

in üblicher Weise zu isolieren. Die neuen Verbindungen der Formeln (IVa) bzw. (IVb) können außerdem in mehreren geometrischen Isomeren der syn- und anti-Formen vorliegen. In allen Fällen entstehen jedoch in der zweiten Verfahrensstufe die Endprodukte der Formel (I).

Die nachfolgenden Beispiele dienen zur näheren Erläuterung der Erfindung.

## Herstellungsbeispiele

### Beispiel 1

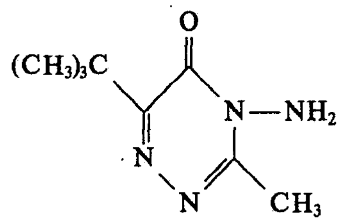

### Beispiel 1a

Zu 12,9 g (0,1 Mol) Trimethylbrenztraubensäureamid, 1000 ml Toluol und 16 g (0,13 Mol) Acethydrazidinhydrochlorid gibt man 1 g p-Toluolsulfonsäure und erhitzt 12 Stunden unter Rückfluß. Danach gibt man 10 g wasserfreies, gemahlenes Kaliumcarbonat zu und erhitzt weitere 4 Stunden unter Rückfluß. Die Lösung wird heiß filtriert, das Filtrat im Vakuum eingeengt und das Produkt unter Zusatz von 20 ml Petrolether auskristallisiert. Nach Filtration und Trocknung bei 80°C im Vakuum erhält man 12,2 g (67% der Theorie) 4-Amino-6-tert.-butyl-3-methyl-1,2,4-triazin-5-on vom Schmelzpunkt 156°C; Schmelzpunkt einer durch Umkristallisieren (aus Benzol) gereinigten Probe 161–163°C.

#### Herstellung des Ausgangsproduktes

$$(CH_3)_3C{-}CO{-}CO{-}NH_2$$

Zu 800 g einer 36%igen Lösung von Bromwasserstoff in Eisessig werden bei Raumtemperatur unter Rühren 111,0 g (1 Mol) Pivaloylcyanid getropft. Nach beendeter Zugabe läßt man 3 Stunden bei Raumtemperatur nachrühren. Danach tropft man bei 20 bis 25°C 9 ml (0,5 Mol) Wasser hinzu und rührt 1 Stunde bei Raumtemperatur nach. Anschließend gießt man die Reaktionslösung in einen Überschuß gesättigter Natriumhydrogencarbonatlösung. Man extrahiert dreimal mit je 200 ml Methylenchlorid. Die vereinigten organischen Phasen werden mit 200 ml Wasser gewaschen, über Natriumsulfat getrocknet und durch Abdestillieren des Lösungsmittels im Vakuum eingeengt. Das zurückbleibende Öl kristallisiert nach einiger Zeit durch. Nach Umkristallisation aus Petrolether erhält man 113,5 g (88% der Theorie) Trimethylbrenztraubensäureamid vom Schmelzpunkt 69–70°C.

### Beispiel 1b

Die Mischung aus 17,1 g (0,1 Mol) Trimethylbrenztraubensäure-N-acetylamid, 12,5 g (0,1 Mol) Acethydrazidinhydrochlorid und 160 ml Methanol wird 1 Stunde bei Raumtemperatur gerührt und

eingeengt. Der Rückstand wird mit 100 ml Pyridin 2 Stunden bei 50°C gerührt und eingedampft. Nach Umkristallisieren aus Ethanol erhält man 15,8 g (87% der Theorie) 4-Amino-6-tert.-butyl-3-methyl-1,2,4-triazin-5-on vom Schmelzpunkt 161–163°C.

Herstellung des Ausgangsproduktes

$(CH_3)_3C-CO-CO-NH-CO-CH_3$

In 49,0 g (0,5 Mol) vorgelegte konzentrierte Schwefelsäure werden jeweils bei Raumtemperatur zunächst 25,6 g (0,25 Mol) Essigsäureanhydrid und anschließend 27,8 g (0,25 Mol) Pivaloylcyanid eingetragen. Nach 4stündigem Nachrühren wird die Reaktionsmischung mit 150 g Eiswasser versetzt und gut durchgerührt. Das ausfallende Reaktionsprodukt wird abgesaugt, mit 100 ml Wasser gewaschen und getrocknet. Man erhält 37,0 g (86,5% der Theorie) Trimethylbrenztraubensäure-N-acetylamid als farblose glänzende Blättchen vom Schmelzpunkt 82–84°C.

Beispiel 1c

15 g (0,12 Mol) Acethydrazidin-hydrochlorid werden in 100 ml Wasser suspendiert und anschließend werden 17,1 g (0,1 Mol) Trimethylbrenztraubensäure-N-acetylamid zugegeben. Man rührt 4 Stunden bei 50°C und stellt die saure Lösung mit 25%iger Ammoniaklösung alkalisch. Das ausfallende Produkt wird bei 20°C abgesaugt und mit Wasser gewaschen. Man erhält 14,3 g (78% der Theorie) 4-Amino-6-tert.-butyl-3-methyl-1,2,4-triazin-5-on als glänzende weiße Blättchen vom Schmelzpunkt 155–157°C.

Beispiel 2

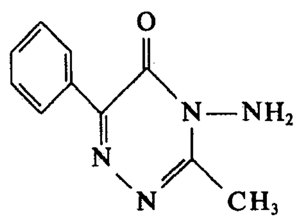

Beispiel 2a

Zu 1,5 g (0,01 Mol) Phenylglyoxylsäureamid, 1,3 g (0,01 Mol) Acethydrazidin-hydrochlorid und 100 ml Toluol gibt man 0,1 g p-Toluolsulfonsäure und erhitzt die Mischung 12 Stunden unter Rühren auf Rückfluß. Anschließend veretzt man mit 10 g wasserfreiem, gemahlenem Kaliumcarbonat und erhitzt weitere 4 Stunden unter Rückfluß. Danach wird heiß filtriert, das Produkt durch Abkühlen des Filtrates auskristallisiert und bei 80°C getrocknet. Man erhält 1,8 g (89% der Theorie) 4-Amino-3-methyl-6-phenyl-1,2,4-triazin-5-on vom Schmelzpunkt 158–161°C.

Beispiel 2b

19,1 g Phenylglyoxylsäure-N-acetylamid (0,1 Mol), 12,5 g (0,1 Mol) Acethydrazidin-hydrochlorid und 160 ml Methanol werden 1 Stunde bei Raumtemperatur gerührt. Das Methanol wird abgedampft und der Rückstand mit 100 ml Pyridin 2 Stunden bei 50°C gerührt. Es wird eingeengt und der Rückstand mit Wasser verrührt. Das Ungelöste wird aus Methanol umkristallisiert. Man erhält 17,9 g (88,6% der Theorie) 4-Amino-3-methyl-6-phenyl-1,2,4-triazin-5-on vom Schmelzpunkt 165–167°C.

Herstellung des Ausgangsproduktes

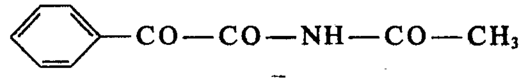

Arbeitet man analog zu Beispiel 1 b/Ausgangsprodukt und setzt anstelle von Pivaloylcyanid Benzoylcyanid ein, so erhält man 38,5 g (79,5% der Theorie) Phenylglyoxylsäure-N-acetylamid als farblose Kristalle vom Schmelzpunkt 124–125°C.

Beispiel 2c

15 g (0,12 Mol) Acethydrazidin-hydrochlorid werden in 100 ml Wasser suspendiert, anschließend werden 19,1 g (0,1 Mol) Phenylglyoxylsäure-N-acetylamid zugegeben. Die Suspension wird 4 Stunden bei 80°C gerührt, mit 25%iger Ammoniaklösung alkalisch gestellt und bei 20°C abgesaugt. Man erhält 16,8 g (83% der Theorie) 4-Amino-3-methyl-6-phenyl-1,2,4-triazin-5-on vom Schmelzpunkt 166—168°C.

In entsprechender Weise und gemäß den angegebenen Verfahrensbeschreibungen können die folgenden Verbindungen der allgemeinen Formel

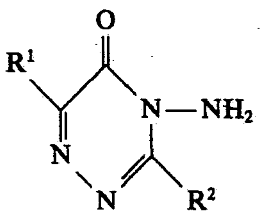

(I)

hergestellt werden.

Tabelle 1

| Beispiel Nr. | $R^1$ | $R^2$ | Schmelzpunkt (°C) |
|---|---|---|---|
| 3 | 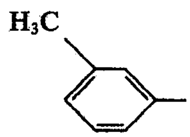 | $CH_3$ | 107 |
| 4 | 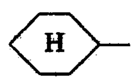 | $CH_3$ | 110 |
| 5 | 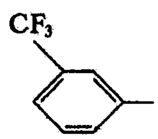 | $CH_3$ | 169 |
| 6 | 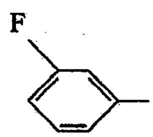 | $CH_3$ | 137 |
| 7 | 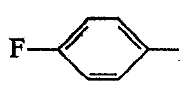 | $CH_3$ | 163 |
| 8 | 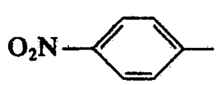 | $CH_3$ | 233 |
| 9 | 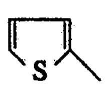 | $CH_3$ | 210 |
| 10 | 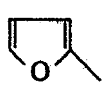 | $CH_3$ | 247 |
| 11 | 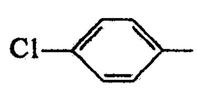 | $C_2H_5$ | 156 |

Fortsetzung

| Beispiel Nr. | $R^1$ | $R^2$ | Schmelzpunkt (°C) |
|---|---|---|---|
| 12 | 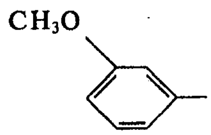 | $C_2H_5$ | 133 |
| 13 | 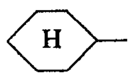 | $C_2H_5$ | 114 |
| 14 | 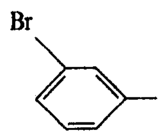 | $C_2H_5$ | 158 |
| 15 | $C_3H_7$-iso | $C_3H_7$-n | 96 |
| 16 | 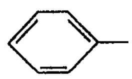 | $C_3H_7$-n | 106 |
| 17 | 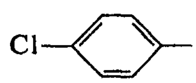 | $C_4H_9$-tert | 153 |
| 18 | 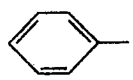 | 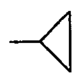 | 121 |
| 19 | $-C(CH_3)_3$ | 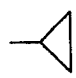 | 110 |
| 20 | $-C(CH_3)_3$ | $-C_2H_5$ | 96 |

**Patentansprüche**

1. Verfahren zur Herstellung von 3,6-disubstituierten 4-Amino-1,2,4-triazin-5-onen der Formel

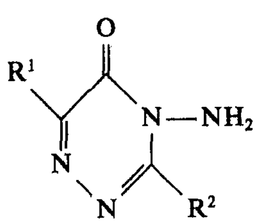

(I)

in welcher

$R^1$ für Wasserstoff, gegebenenfalls substituiertes Alkyl, gebenenenfalls substituiertes Cycloalkyl, gegebenenfalls substituiertes Cycloalkenyl, Alkenyl, Furyl, Thienyl oder gegebenenfalls substituiertes Aralkyl steht und
$R^2$ für Alkyl, gegebenenfalls substituiertes Cycloalkyl, gegebenenfalls substituiertes Aryl oder gegebenenfalls substituiertes Aralkyl steht,

dadurch gekennzeichnet, daß man $\alpha$-Ketocarbonsäureamide der Formel

$$R^1-CO-CO-NHR^3 \quad (II)$$

in welcher

$R^1$ die oben angegebene Bedeutung hat und
$R^3$ für Wasserstoff oder die Gruppe $-CO-R^4$ steht, wobei
$R^4$ für Alkyl, Cycloalkyl, gegebenenfalls substituiertes Aryl oder gegebenenfalls substituiertes Aralkyl steht,

pro Mol mit 1–1,5 Mol eines Hydrazidins der Formel

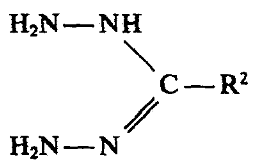
(III)

in welcher

$R^2$ die oben angegebene Bedeutung hat,

oder eines ihrer Säureadditionssalze, gegebenenfalls in Gegenwart eines Verdünnungsmittels, bei Temperaturen zwischen 0° und 150°C umsetzt, wobei in der ersten Verfahrensstufe eine Kondensationsreaktion zu noch offenkettigen Kondensationsprodukten und in der zweiten Verfahrensstufe eine Eliminierungsreaktion unter Ringschluß zu den Endprodukten (I) stattfindet.

2. Verfahren nach Anspruch 1, dadurch gekennzeichnet, daß die erste Verfahrensstufe in Gegenwart eines sauren Katalysators durchgeführt wird.

3. Verfahren nach Anspruch 1, dadurch gekennzeichnet, daß man die zweite Verfahrensstufe in Gegenwart einer Base durchführt.

4. Verfahren nach Anspruch 1, dadurch gekennzeichnet, daß die zweite Verfahrensstufe ohne Zwischenisolierung der in der ersten Verfahrensstufe gebildeten Kondensationsprodukte durchgeführt wird.

5. Verfahren nach Anspruch 1, dadurch gekennzeichnet, daß man bei Temperaturen zwischen 10 und 120°C arbeitet.

6. Verfahren nach Anspruch 1, dadurch gekennzeichnet, daß man auf 1 Mol $\alpha$-Ketocarbonsäureamid der Formel (II) 1–1,3 Mol eines Hydrazidins der Formel (III) bzw. eines entsprechenden Hydrazidin-Säureadditionssalzes und in der ersten Verfahrensstufe gegebenenfalls 0,05–0,1 Mol eines sauren Katalysators sowie in der zweiten Verfahrensstufe gegebenenfalls 1–3,5 Mol einer Base einsetzt.

7. Verfahren zur Herstellung von 3,6-disubstituierten 4-Amino-1,2,4-triazin-5-onen der Formel (I), in welcher

$R^1$ für Isopropyl, sek.-Butyl, n-Butyl, tert.-Butyl, sek.-Pentyl, Neopentyl, 1,1-Dimethylpropyl, Dichlorcyclopropyl, Methyldichlorcyclopropyl, Cyclobutyl, Cyclopentyl, Cyclohexyl, Furyl, Thienyl, sowie für gegebenenfalls durch Fluor, Chlor, Brom, Methoxy, Nitro, Methyl und Trifluormethyl substituiertes Phenyl steht und
$R^2$ für Methyl, Ethyl, n-Propyl, Isopropyl, n-Butyl, sek.-Butyl, tert.-Butyl, Cyclopropyl, Cyclobutyl oder Cyclopentyl steht,

gemäß Anspruch 1.

## Claims

1. Process for the preparation of 3,6-disubstituted 4-amino-1,2,4,-triazin-5-ones of the formula

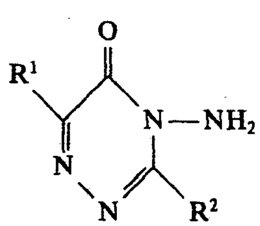
(I)

in which

$R^1$ represents hydrogen, optionally substituted alkyl, optionally substituted cycloalkyl, optionally substituted cycloalkenyl, alkenyl, furyl, thienyl or optionally substituted aralkyl, and

$R^2$ represents alkyl, optionally substituted cycloalkyl, optionally substituted aryl or optionally substituted aralkyl,

characterised in that $\alpha$-ketocarboxylic acid amides of the formula

$$R^1—CO—CO—NHR^3 \qquad (II)$$

in which

$R^1$ has the meaning given above, and
$R^3$ represents hydrogen or the group $-CO-R^4$, wherein
$R^4$ represents alkyl, cycloalkyl, optionally substituted aryl or optionally substituted aralkyl,

are reacted, per mol, with 1—1.5 mols of a hydrazidine of the formula

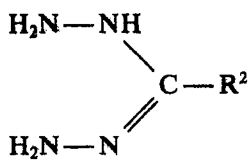

(III)

in which

$R^2$ has the meaning given above,

or one of their acid addition salts, if appropriate in the presence of a diluent at témperatures between 0 and 150°C, whereby in the first process stage a condensation reaction takes place to give condensation products which still have open chains, and in the second process stage an elimination reaction with ring closure takes place to give the end products (I).

2. Process according to Claim 1, characterised in that the first process stage is carried out in the presence of an acid catalyst.

3. Process according to Claim 1, characterised in that the second process stage is carried out in the presence of a base.

4. Process according to Claim 1, characterised in that the second process stage is carried out without intermediate isolation of the condensation products formed in the first process stage.

5. Process according to Claim 1, characterised in that the reaction is carried out at temperatures between 10 and 120°C.

6. Process according to Claim 1, characterised in that 1 to 1.3 mol of a hydrazidine of the formula (III) or an appropriate hydrazidine acid addition salt, and in the first process stage, if appropriate, 0.05 to 0.1 mol of an acid catalyst and in the second process stage, if appropriate, 1 to 3.5 mol of a base, are employed per mol of $\alpha$-ketocarboxylic acid amide of the formula (II).

7. Process for the preparation of 3,6-disubstituted 4-amino-1,2,4-triazin-5-ones of the formula (I), in which

$R^1$ represents isopropyl, sec.-butyl, n-butyl, tert.-butyl, sec.-pentyl, neopentyl, l,l-dimethylpropyl, dichlorocyclopropyl, methyldichlorocyclopropyl, cyclobutyl, cyclopentyl, cyclohexyl, furyl, thienyl, and phenyl which is optionally substituted by fluorine, chlorine, bromine, methoxy, nitro, methyl and trifluoromethyl, and
$R^2$ represents methyl, ethyl, n-propyl, isopropyl, n-butyl, sec.-butyl, tert.-butyl, cyclopropyl, cyclobutyl or cyclopentyl,

according to Claim 1.

**Revendications**

1. Procédé de production de 4-amino-1,2,4-triazine-5-ones disubstituées en positions 3, 6, de formule

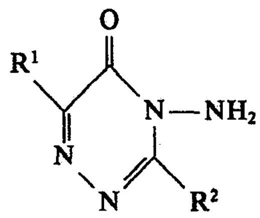

(I)

dans laquelle

$R^1$ représente l'hydrogène, un groupe alkyle éventuellement substitué, cycloalkyle éventuellement substitué, cycloalcényle éventuellement substitué, alcényle, furyle, thiényle ou aralkyle éventuellement substitué et

$R^2$ est un groupe alkyle, cycloalkyle éventuellement substitué, aryle éventuellement substitué ou aralkyle éventuellement substitué,

caractérisé en ce qu'on fait réagir des amides d'acides $\alpha$-cétocarboxyliques de formule

$$R^1—CO—CO—NHR^3 \qquad (II)$$

dans laquelle

$R^1$ a la définition indiquée ci-dessus et

$R^3$ est l'hydrogène ou le groupe $-CO-R^4$

$R^4$ représentant un groupe alkyle, cycloalkyle, aryle éventuellement substitué ou aralkyle éventuellement substitué,

par mole, avec 1–1,5 mole d'une hydrazidine de formule

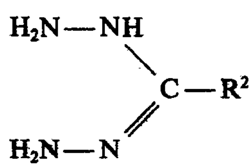

(III)

dans laquelle

$R^2$ a la définition indiquée ci-dessus,

ou de l'un de ses sels d'addition d'acides, éventuellement en présence d'un diluant, à des températures comprises entre 0 et 150°C, et il se produit alors dans la première étape du procédé une réaction de condensation en produits de condensation à chaîne encore ouverte et dans la seconde étape du procédé, une réaction d'élimination avec cyclisation en les produits finals (I).

2. Procédé suivant la revendication 1, caractérisé en ce que la première étape du procédé est conduite en présence d'un catalyseur acide.

3. Procédé suivant la revendication 1, caractérisé en ce qu'on conduit la seconde étape du procédé en présence d'une base.

4. Procédé suivant la revendication 1, caractérisé en ce que la seconde étape du procédé est conduite sans isolement intermédiaire des produits de condensation formés dans la première étape.

5. Procédé suivant la revendication 1, caractérisé en ce qu'on opère à des températures comprises entre 10 et 120°C.

6. Procédé suivant la revendication 1, caractérisé en ce qu'on utilise par mole d'amide d'acide $\alpha$-cétocarboxylique de formule (II) 1 à 1,3 mole d'une hydrazidine de formule (III) ou d'un sel d'addition d'acide d'hydrazidine correspondant et on utilise dans la première étape du procédé, le cas échéant, 0,05 à 0,1 mole d'un catalyseur acide ainsi que dans la seconde étape, le cas échéant, 1 à 3,5 moles d'une base.

7. Procédé de production de 4-amino-1,2,4-triazine-5-ones disubstituées en positions 3, 6 de formule (I), dans laquelle

$R^1$ est un groupe isopropyle, butyle secondaire, n-butyle, tertio-butyle, pentyle secondaire, néo-pentyle, 1,1-diméthylpropyle, dichlorocyclopropyle, méthyldichlorocyclopropyle, cyclobutyle, cyclopentyle, cyclohexyle, furyle, thiényle ainsi qu'un groupe phényle éventuellement substitué par du fluor, du chlore, du brome, les radicaux méthoxy, nitro, méthyle et trifluorométhyle et

$R^2$ est un groupe méthyle, éthyle, n-propyle, isopropyle, n-butyle, butyle secondaire, tertio-butyle, cyclopropyle, cyclobutyle ou cyclopentyle.